# EUROPEAN PATENT APPLICATION

(11) **EP 2 532 655 A1**
(43) Date of publication of application: **12.12.2012**
(21) Application number: 11739854.5
(22) Date of filing: 04.02.2011
(51) Int. Cl.: C07D 307/91, C09K 11/06, H01L 51/50

(54) **AMINOANTHRACENE DERIVATIVE AND ORGANIC ELECTROLUMINESCENT ELEMENT FORMED USING SAME**

(30) Priority: 05.02.2010 JP 2010024764
(71) Applicant: Idemitsu Kosan Co., Ltd., Chiyoda-ku Tokyo 100-8321 (JP)
(72) Inventor: MIZUTANI, Sayaka, Sodegaura-shi Chiba 299-0293 (JP); SADO, Takayasu, Sodegaura-shi Chiba 299-0293 (JP)
(74) Representative: Weinberger, Rudolf
(86) International application number: PCT/JP2011/052331
(87) International publication number: WO 2011/096506

(57) **Abstract**

There are provided an aminoanthracene derivative having an end substituent, such as a dibenzofuran ring, that is bonded at a position other than the 2-position in the formula (1) to anthracene through a nitrogen atom, and an organic electroluminescent device containing a cathode and an anode, and therebetween one or plural organic thin film layers containing at least a light emitting layer, at least one layer of the organic thin film layers containing the aminoanthracene derivative. The organic electroluminescent device has a long lifetime and a high luminous efficiency. (In the formula, R₁, m and Ar₁ to Ar₄ have the meanings described in Claim 1.)

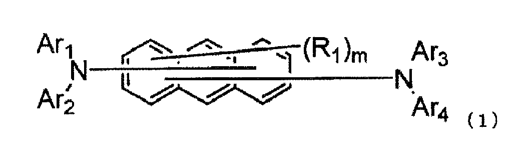

## Description

### Technical Field

The present invention relates to an aminoanthracene derivative and an organic electroluminescent device using the same, and more particularly, relates to an organic electroluminescent device that has a long lifetime and a high luminous efficiency, and an aminoanthracene derivative that realizes the same.

### Background Art

An organic electroluminescent (EL) device using an organic substance is being promised for the purpose of an inexpensive large-area full color display device of a solid state light emission type, and is being developed variously. In general, the organic EL device is constituted by a light emitting layer and a pair of opposing electrodes holding the layer. Upon emitting light, an electric field is applied between the electrodes, whereby electrons are injected from the cathode, and positive holes are injected from the anode. The electrons are recombined with the positive holes in the light emitting layer to form an excited state, and energy is discharged as light upon returning the excited state to the ground state.
The ordinary organic EL device requires a high driving voltage and exhibits low light emission luminance and low luminous efficiency, as compared to an inorganic light emitting diode. The ordinary organic EL device also exhibits remarkable deterioration of characteristics, and thus is still insufficient for practical use. The organic EL devices in recent years are improved little by little, but are demanded to have a higher luminous efficiency and a longer lifetime.
For example, there is disclosed a technique using a sole monoanthracene compound as an organic light emitting material (Patent Document 1). In the technique, however, for example, only a luminance of 1,650 cd/m² is obtained at a current density of 165 mA/cm², and thus the efficiency is 1 cd/A, which is considerably low and is not suitable for practical use. There is disclosed a technique using a sole bisanthracene compound as an organic light emitting material (Patent Document 2). In the technique, however, the efficiency is as low as from 1 to 3 cd/A, and there is a demand of improvement for practical use. There is proposed an organic EL device having a long lifetime using a distyryl compound as an organic light emitting material, to which styrylamine or the like is added (Patent Document 3). However, the device is insufficient in lifetime and is demanded to be further improved.
There is disclosed a technique using a mono- or bisanthracene compound and a distyryl compound as an organic light emitting layer (Patent Document 4). In the technique, however, the light emitting spectrum is shifted to the longer wavelength due to the conjugated structure of the styryl compound, which deteriorates the color purity.
Patent Document 5 discloses an invention using an aromatic amine derivative having an arylene group as the center and a dibenzofuran ring bonded to the nitrogen atom, as a positive hole transporting material, and Patent Document 6 discloses an invention using an aromatic amine derivative having a dibenzofuran ring, a dibenzothiophene ring, a benzofuran ring, a benzothiophene ring or the like bonded to the nitrogen atom through an arylene group, as a positive hole transporting compound. However, there is no case of using as a light emitting material.
Patent Document 7 includes a disclosure of anthracene having a dibenzofuranylamino group. However, there is a demand of further improvement of the luminous efficiency and the lifetime.

### Related Art Documents

### Patent Documents

Patent Document 1: JP-A-11-3782
Patent Document 2: JP-A-8-12600
Patent Document 3: WO94/00615
Patent Document 4: JP-A-2001-284050
Patent Document 5: Japanese Patent No. 3,508,984
Patent Document 6: WO2007/12571
Patent Document 7: WO2009/08451

### Summary of the Invention

### Problems to be solved by the Invention

The present invention has been made for solving the problems, and an object thereof is to provide an organic EL device that has a long lifetime and a high luminous efficiency, and an aminoanthracene derivative that achieving the same.

### Means for solving the Problems

As a result of earnest investigations made by the inventors, it has been found that a high luminous efficiency and a long lifetime are obtained by using an aminoanthracene derivative having an end substituent, such as a dibenzofuran ring or a dibenzothiophene ring, that is bonded at a position other than the 2-position, as an organic EL device material, and thus the present invention has been completed.
The present invention thus provides an aminoanthracene derivative represented by the following formula (1):

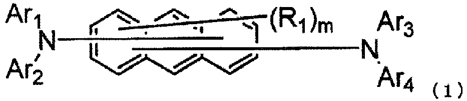

wherein R₁ represents a hydrogen atom, a halogen atom (preferably a fluorine atom), a substituted or unsubstituted aryl group having 6 to 50 ring carbon atoms, a substituted or unsubstituted a heterocyclic group having 5 to 50 ring atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 7 to 50 ring carbon atoms, a substituted or unsubstituted aryloxy group having 6 to 50 ring carbon atoms, a substituted or unsubstituted arylthio group having 6 to 50 ring carbon atoms, a substituted or unsubstituted alkoxycarbonyl group having 2 to 50 carbon atoms, a substituted or unsubstituted aryl- and/or alkylsilyl group, a carboxyl group, an amino group, a cyano group, a nitro group or a hydroxyl group; m represents an integer of from 1 to 8, provided that when m is 2 or more, plural groups of R₁ may be the same as or different from each other;
Ar₁ to Ar₄ each independently represent a substituted or unsubstituted aryl group having 6 to 50 ring carbon atoms, a substituted or unsubstituted alkyl group having 1 to 50 carbon atoms, a substituted or unsubstituted cycloalkyl group having 3 to 50 ring carbon atoms, a substituted or unsubstituted aralkyl group having 7 to 50 ring carbon atoms, or a substituted or unsubstituted heterocyclic group having 5 to 50 ring atoms,
provided that one or more of Ar₁ to Ar₄ represents a group represented by the following formula (2), (3) or (4):

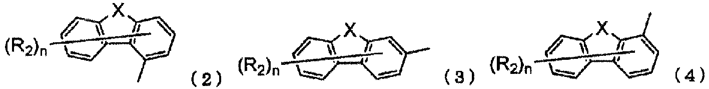

wherein X represents oxygen (O), sulfur (S) or selenium (Se); R₂ represents a hydrogen atom, a substituted or unsubstituted aryl group having 6 to 60 ring carbon atoms, a substituted or unsubstituted alkyl group having 1 to 50 carbon atoms, a substituted or unsubstituted alkenyl group having 2 to 50 carbon atoms, an amino group, a substituted or unsubstituted alkoxy group having 1 to 50 carbon atoms, a substituted or unsubstituted aryloxy group having 6 to 50 ring carbon atoms, a substituted or unsubstituted arylthio group having 6 to 50 ring carbon atoms, a substituted or unsubstituted alkoxycarbonyl group having 1 to 50 carbon atoms, a halogen atom (preferably a fluorine atom), a cyano group, a nitro group, a hydroxyl group, a substituted or unsubstituted aryl- and/or alkylsilyl group, or a carboxyl group; and n represents an integer of from 1 to 7, provided that when n is 2 or more, plural groups of R₂ may be the same as or different from each other; when there are plural groups of R₂, the groups may be bonded to form a saturated or unsaturated divalent group, which may be substituted; and a case where R₂ is bonded to the 5-membered ring moiety containing X is excluded.

The present invention also provides an organic EL device containing a cathode and an anode, and therebetween one or plural organic thin film layers containing at least a light emitting layer, at least one layer of the organic thin film layers containing the aminoanthracene derivative solely or as a component of a mixture.

### Advantages of the Invention

The organic EL device using the aminoanthracene derivative of the present invention has a high luminous efficiency and is hard to be deteriorated to have a long lifetime.

### Embodiments for carrying out the Invention

The anthracene compound of the present invention is a compound represented by the following formula (1):

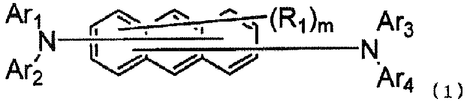

In the formula (1), R₁ represents a hydrogen atom, a halogen atom (preferably a fluorine atom), a substituted or unsubstituted aryl group having 6 to 50 ring carbon atoms (preferably having 6 to 20 ring carbon atoms), a substituted or unsubstituted a heterocyclic group having 5 to 50 ring atoms (preferably having 5 to 20 ring atoms), a substituted or unsubstituted alkyl group having 1 to 50 carbon atoms (preferably from 1 to 20 carbon atoms), a substituted or unsubstituted cycloalkyl group having 3 to 50 carbon atoms (preferably having 5 to 12 ring carbon atoms), a substituted or unsubstituted alkoxy group having 1 to 50 carbon atoms (preferably from 1 to 20 carbon atoms), a substituted or unsubstituted aralkyl group having 7 to 50 ring carbon atoms (preferably having 7 to 20 ring carbon atoms), a substituted or unsubstituted aryloxy group having 6 to 50 ring carbon atoms (preferably having 7 to 20 ring carbon atoms), a substituted or unsubstituted arylthio group having 6 to 50 ring carbon atoms (preferably having 7 to 20 ring carbon atoms), a substituted or unsubstituted alkoxycarbonyl group having 2 to 50 carbon atoms (preferably from 2 to 20 carbon atoms), a substituted or unsubstituted aryl- and/or alkylsilyl group, a carboxyl group, an amino group, a cyano group, a nitro group or a hydroxyl group. m represents an integer of from 1 to 8 (preferably from 1 to 4, and more preferably from 1 to 2), provided that when m is 2 or more, plural groups of R₁ may be the same as or different from each other.

Specific examples of the groups of R₁ will be described below.
Examples of the aryl group include a phenyl group, a 2-methylphenyl group, a 3-methylphenyl group, a 4-methylphenyl group, a 4-ethylphenyl group, a biphenylyl group, a 4-methylbiphenylyl group, a 4-ethylbiphenylyl group, 4-cyclohexylbiphenylyl group, a terphenylyl group, 3,5-dichlorophenyl group, a naphthyl group, a 5-methylnaphthyl group, a phenanthryl group, an anthryl group, a pyrenyl group, a chrysenyl group, a foluorantenyl group, a perylenyl group and a 9,9-dimethylfluorenyl group.
Examples of the heterocyclic group include residual groups of imidazole, benzoimidazole, pyrrole, furan, thiophene, oxadiazoline, indoline, carbazole, pyridine, quinoline, isoquinoline, benzoquinone, pyralozine, imidazolidine, piperidine, pyrimidine, benzofuran, dibenzofuran, benzothiophene, dibenzothiophene and triazine.
Examples of the alkyl group include a linear or branched alkyl group having 1 to 20 carbon atoms and a linear or branched haloalkyl group having 1 to 20 carbon atoms, and examples thereof include a methyl group, an ethyl group, a propyl group, an isopropyl group, a butyl group, a sec-butyl group, a tert-butyl group, a pentyl group, a hexyl group, a heptyl group, an octyl group, a stearyl group, a 2-phenylisopropyl group, a trichloromethyl group, a trifluoromethyl group, a benzyl group, an α-phenoxybenzyl group, an α,α-dimethylbenzyl group, an α,α-methylphenylbenzyl group, an α,α-ditrifluoromethylbenzyl group, a triphenylmethyl group and an α-benzyloxybenzyl group.

Examples of the cycloalkyl group include a cyclopropyl group, a cyclobutyl group, a cyclopentyl group, a cyclohexyl group, a cycloheptyl group, a cyclooctyl group, a cyclononyl group, a bicycloheptyl group, a bicyclooctyl group, a tricycloheptyl group and an adamantyl group, and a cyclopentyl group, a cyclohexyl group, a cycloheptyl group, a bicycloheptyl group, a bicyclooctyl group and an adamantyl group are preferred.
Examples of the alkoxy group include a methoxy group, an ethoxy group, a propoxy group, a isopropoxy group, a butoxy group, an isobutoxy group, a sec-butoxy group, a tert-butoxy group, various kinds of pentyloxy groups and various kinds of hexyloxy groups.
Examples of the aralkyl group include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2**-**β**-**naphthylethyl group, a 1-β-naphthylisopropyl group, 2-β-naphthylisopropyl group, a 1-pyrrolylmethyl group, a 2-(1-pyrrolyl)ethyl group, a p-methylbenzyl group, a m-methylbenzyl group, an o-methylbenzyl group, a p-chlorobenzyl group, a m-chlorobenzyl group, an o-chlorobenzyl group, a p-bromobenzyl group, a m-bromobenzyl group, an o-bromobenzyl group, a p-iodobenzyl group, a m-iodobenzyl group, an o-iodobenzyl group, a p-hydroxybenzyl group, a m-hydroxybenzyl group, an o-hydroxybenzyl group, a p-aminobenzyl group, a m-aminobenzyl group, an o-aminobenzyl group, a p-nitrobenzyl group, a m-nitrobenzyl group, an o-nitrobenzyl group, a p-cyanobenzyl group, a m-cyanobenzyl group, an o-cyanobenzyl group, a 1-hydroxy-2-phenylisopropyl group and a 1-chlor-2-phenylisopropyl group.
Examples of the aryloxy group and the arylthio group include groups represented by -OY or -SY, wherein examples of the group of Y include those exemplified above for the aryl group.
Examples of the alkoxycarbonyl group include groups represented by -COOZ, wherein examples of the group of Z include those exemplified above for the alkyl group.
Examples of the aryl- and/or alkylsilyl group include a trimethylsilyl group, a triethylsilyl group, a triphenylsilyl group, a tert-butyldimethylsilyl group and a tert-butyldiphenylsilyl group.

In the formula (1), Ar₁ to Ar₄ each independently represent a substituted or unsubstituted aryl group having 6 to 50 ring carbon atoms (preferably having 6 to 20 ring carbon atoms), a substituted or unsubstituted alkyl group having 1 to 50 carbon atoms (preferably having 1 to 20 carbon atoms), a substituted or unsubstituted cycloalkyl group having 3 to 50 ring carbon atoms (preferably having 5 to 12 ring carbon atoms), a substituted or unsubstituted aralkyl group having 7 to 50 ring carbon atoms (preferably having 7 to 20 ring carbon atoms), or a substituted or unsubstituted heterocyclic group having 5 to 50 ring atoms (preferably having 5 to 20 ring atoms).
Specific examples of the groups represented by Ar₁ to Ar₄ include those exemplified above for R₁.
One or more of Ar₁ to Ar₄ represents a group represented by the following formula (2), (3) or (4).
It is considered that the use of the aminoanthracene derivative that has a rigid and sterically bulky end substituent, such as a dibenzofuran ring or a dibenzothiophene ring bonded at the 1-position, 3-position or 4-position, as a material for an organic EL device enhances the intramolecular steric hindrance, thereby preventing intermolecular association, which provides a high luminous efficiency through prevention of concentration quenching, and a long lifetime.

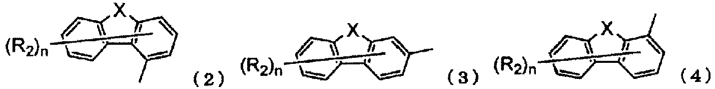

In the formulae (2) to (4), X represents oxygen (O) sulfur (S) or selenium (Se); R₂ represents a hydrogen atom, a substituted or unsubstituted aryl group having 6 to 60 ring carbon atoms (preferably having 6 to 20 carbon atoms), a substituted or unsubstituted alkyl group having 1 to 50 carbon atoms (preferably having 1 to 20 carbon atoms), a substituted or unsubstituted alkenyl group having 2 to 50 carbon atoms (preferably having 2 to 20 carbon atoms), an amino group, a substituted or unsubstituted alkoxy group having 1 to 50 carbon atoms (preferably having 1 to 20 carbon atoms), a substituted or unsubstituted aryloxy group having 6 to 50 ring carbon atoms (preferably having 7 to 20 ring carbon atoms), a substituted or unsubstituted arylthio group having 6 to 50 ring carbon atoms (preferably having 7 to 20 ring carbon atoms), a substituted or unsubstituted alkoxycarbonyl group having 1 to 50 carbon atoms (preferably having 2 to 20 carbon atoms), a halogen atom (preferably a fluorine atom), a cyano group, a nitro group, a hydroxyl group, a substituted or unsubstituted aryl- and/or alkylsilyl group, or a carboxyl group. n represents an integer of from 1 to 7 (preferably from 1 to 4), provided that when n is 2 or more, plural groups of R₂ may be the same as or different from each other. X particularly preferably represents oxygen (O) among oxygen (O), sulfur (S) or selenium (Se).
When there are plural groups of R₂, the groups may be bonded to form a substituted or unsubstituted divalent group, which may be substituted. A case where R₂ is bonded to the 5-membered ring moiety containing X is excluded.
Specific examples of the groups represented by R₂ include those exemplified above for R₁, and examples of the alkenyl group, which is not included in R₁, include a vinyl group, an allyl group, a 1-butenyl group, a 2-butenyl group, a 3-butenyl group, a 1,3-butanedienyl group, a 1-methylvinyl group, a styryl group, a 2,2-diphenylvinyl group and a 1,2-diphenylvinyl group.
Examples of the divalent group formed by bonding the plural groups of R₂ include the following structures where X is oxygen or sulfur, for example, and the divalent group is not limited to these examples. The similar examples may be exemplified in the case where X is an atom other than oxygen and sulfur.

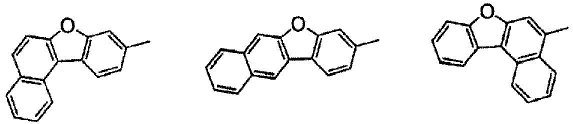

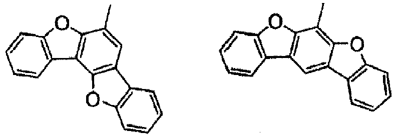

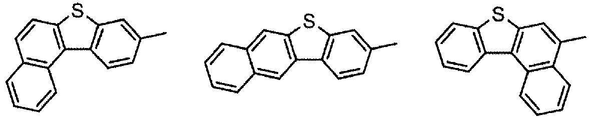

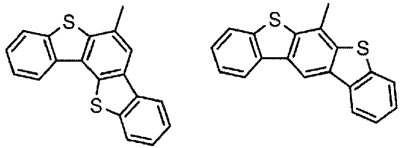

In the "substituted or unsubstituted groups" in the descriptions for the formulae described hereinabove and hereinbelow, examples of the arbitrary substituent include a substituted or unsubstituted aryl group having 6 to 50 ring carbon atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 ring carbon atoms, a substituted or unsubstituted aryloxy group having 6 to 50 ring carbon atoms, a substituted or unsubstituted arylthio group having 6 to 50 ring carbon atoms, a substituted or unsbstituted alkoxycarbonyl group having 1 to 50 carbon atoms, an amino group, a halogen atom (preferably a fluorine atom), a substituted or unsubstituted aryl- and/or alkylsilyl group, a cyano group, a nitro group, a hydroxyl group and a carboxyl group.
Among these, an alkyl group having 1 to 10 carbon atoms, a cycloalkyl group having 5 to 7 carbon atoms and an alkoxy group having 1 to 10 carbon atoms are preferred, an alkyl group having 1 to 6 carbon atoms and a cycloalkyl group having 5 to 7 carbon atoms are more preferred, and a methyl group, an ethyl group, a n-propyl group, an isopropyl group, a n-butyl group, a sec-butyl group, a tert-butyl group, a n-pentyl group, a n-hexyl group, a cyclopentyl group and a cyclohexyl group are particularly preferred.
In the present invention, the "aryl group" means a "group derived from an aromatic compound by removing a hydrogen atom thereof", and includes not only a monovalent aryl group, but also an "arylene group", which is divalent.
The term "unsubstituted" means that a hydrogen atom is substituted.
The hydrogen atom of the compounds described in the present specification includes light hydrogen and deuterium.

In the formula (1), Ar₁ and Ar₃ are each preferably a group represented by the formula (2), (3) or (4), and more preferably a group represented by the formula (4).
The formula (1) preferably represents the following formula (6):

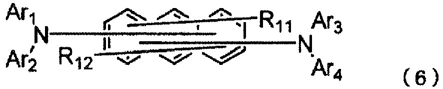

1 wherein Ar₁ to Ar₄ have the same meanings as above; and R₁₁ and R₁₂ each have the same meaning as R₁.
It is preferred that -NAr₁Ar₂ is bonded to the 2-position of the anthracenylene group in the formula (6), and -Nar₃Ar₄ is bonded to the 6-position thereof, or in alternative, -NAr₁Ar₂ is bonded to the 9-position of the anthracenylene group in the formula (6), and -Nar₃Ar₄ is bonded to the 10-position thereof.

Specific examples of the aminoanthracene derivative represented by the formula (1) of the present invention are shown below, but the present invention is not limited to the examples.

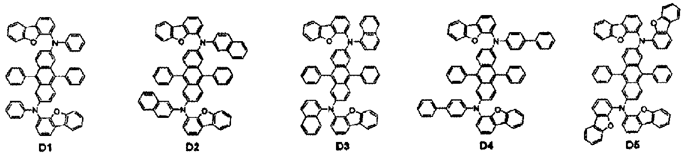

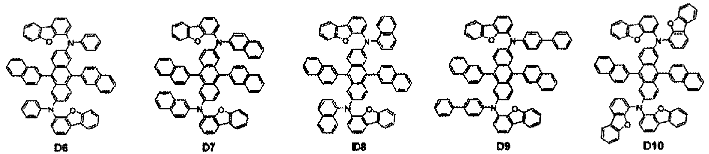

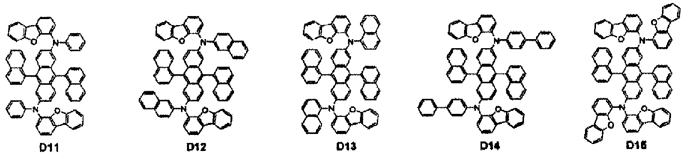

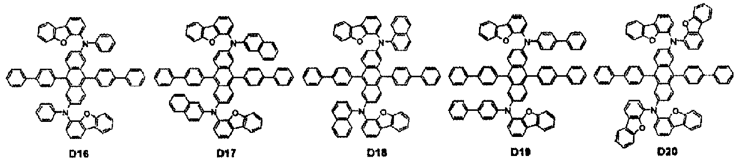

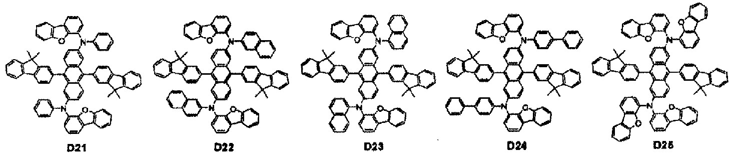

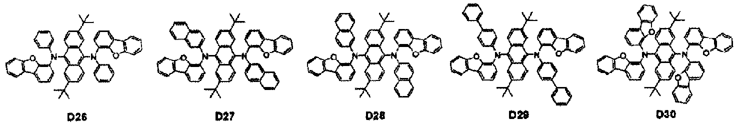

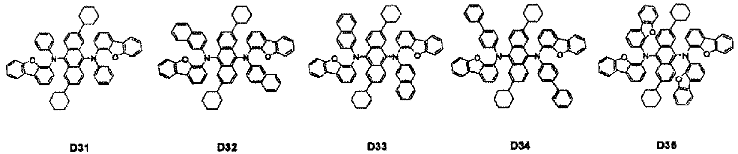

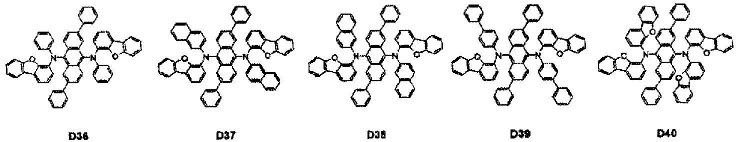

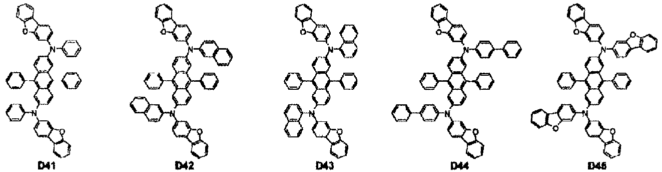

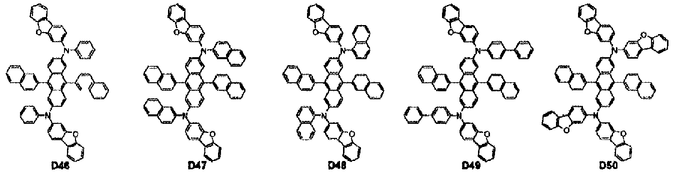

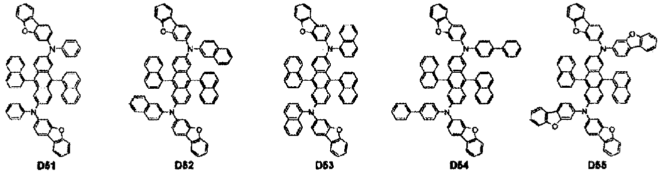

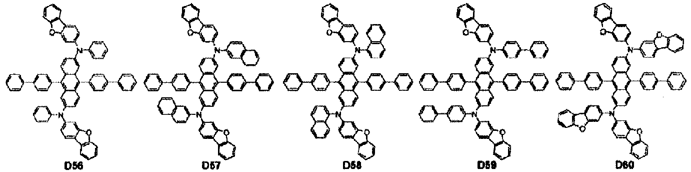

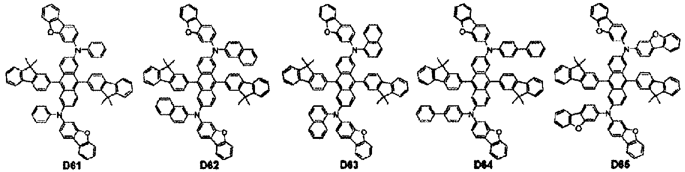

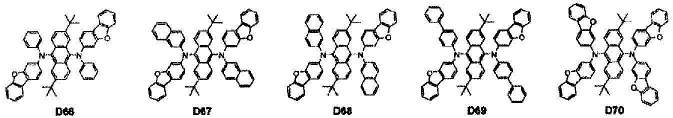

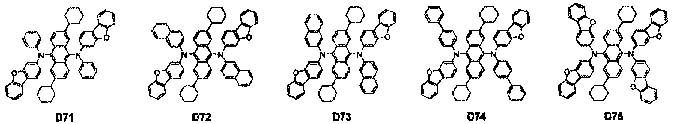

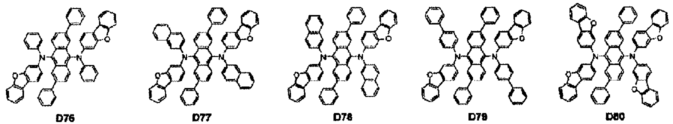

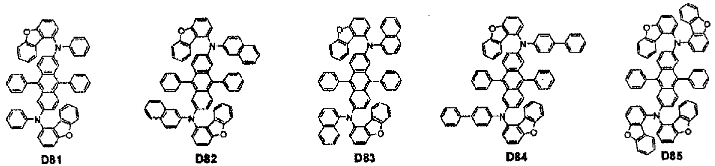

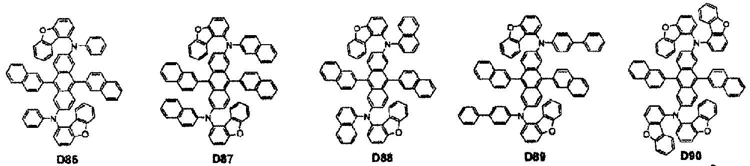

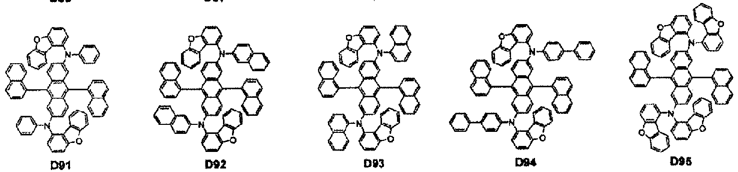

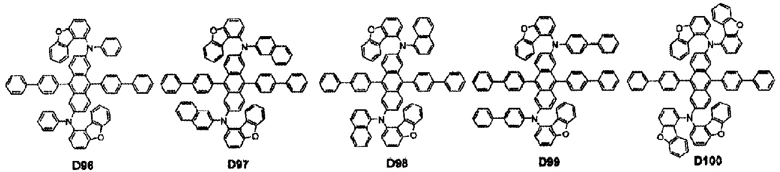

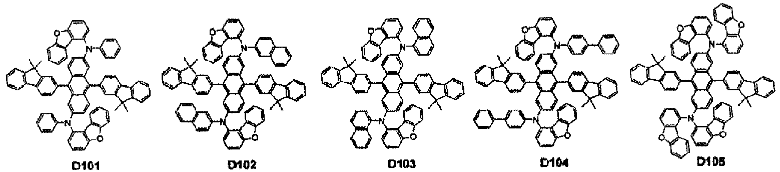

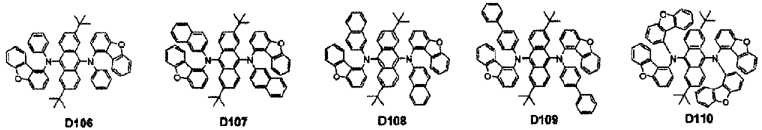

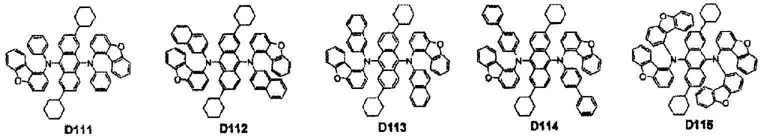

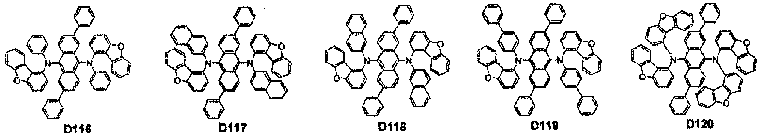

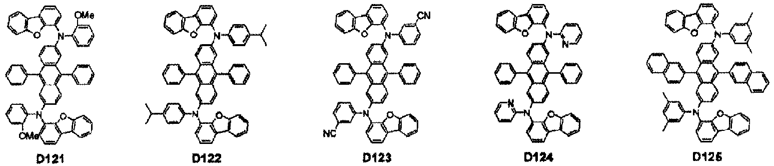

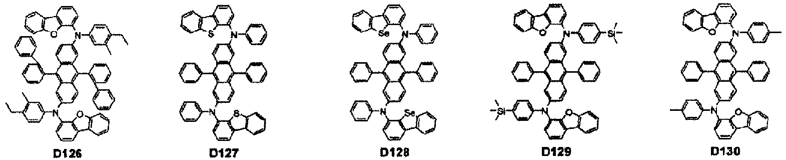

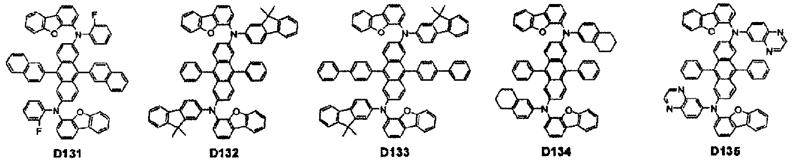

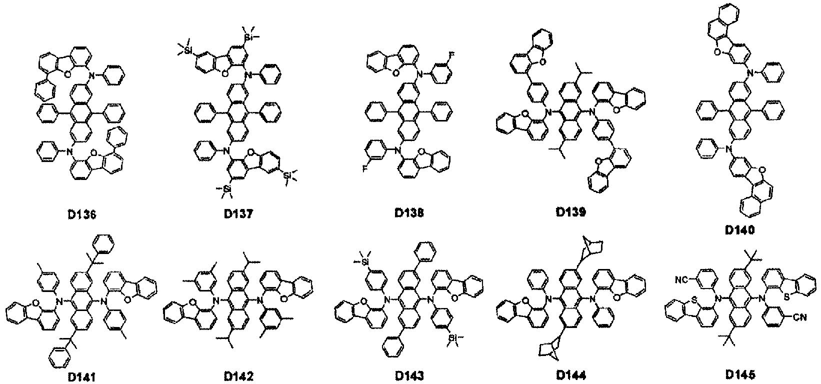

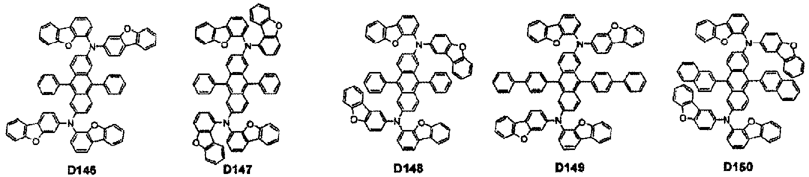

A production method of the aminoanthracene derivative of the present invention will be described.
The production method of the aminoanthracene derivative represented by the formula (1) of the present invention is not particularly limited, and the derivative may be produced by a known method. For example, 2,6-dibromoanthraquinone obtained by the method described in Chem. Commun., 1997, 73 (M.A. Rabjohns, et al.) may be aminated with diarylamine, thereby producing an aromatic amine derivative.

The aminoanthracene derivative of the present invention is favorably used as a material for an organic EL device, and in particular, the derivative is favorably used as a light emitting material, and preferably a blue light emitting material or a green light emitting material.
The aminoanthracene derivative of the present invention is also favorably used as a doping material for an organic EL device.

The organic EL device of the present invention contains an anode and a cathode, and therebetween one or plural organic thin film layers. In the case of the single layer type, a light emitting layer is provided between the anode and the cathode. The light emitting layer contains a light emitting material and may further contain a positive hole injection material or an electron transporting material for transporting positive holes injected from the anode or electrons injected from the cathode, to the light emitting material. The aminoanthracene derivative of the present invention has high light emitting characteristics and is excellent in positive hole injection property, positive hole transporting characteristics, electron injection property and electron transporting characteristics, and therefore, the derivative may be favorably used as a light emitting material or a doping material in a light emitting layer.
In the organic EL device of the present invention, the light emitting layer preferably contains the aminoanthracene derivative of the present invention, and the content thereof is generally from 0.1 to 20% by weight, and more preferably from 1 to 10% by weight. The aminoanthracene derivative of the present invention has both considerably high fluorescent quantum efficiency and high positive hole transporting capability and electron transporting capability, and can be formed into a uniform thin film, and therefore, the light emitting layer can be formed with the aminoanthracene derivative solely.

In the case where the aminoanthracene derivative of the present invention is used as a light emitting material of an organic EL device, the light emitting layer preferably contains at least one kind of the aminoanthracene derivative of the present invention and at least one kind selected from compounds represented by the following formula (40). The at least one kind selected from the aminoanthracene derivative of the present invention is preferably a doping material, and the at least one kind selected from compounds represented by the following formula (40) is preferably a host material.
The compound represented by the formula (40) will be described below.

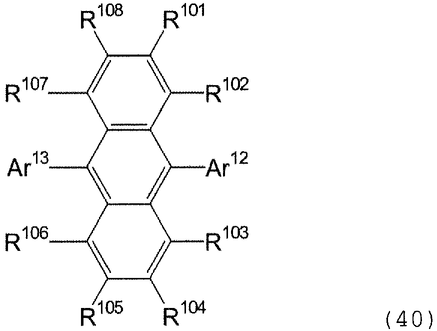

wherein in the formula (40), R¹⁰¹ to R¹⁰⁸ each represent a hydrogen atom, a fluorine atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 30 carbon atoms, a substituted or unsubstituted arylsilyl group having 8 to 30 ring carbon atoms, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon atoms, or a substituted or unsubstituted heterocyclic group having 5 to 30 ring atoms; and Ar¹² and Ar¹³ each represent a substituted or unsubstituted aryl group having 6 to 30 ring carbon atoms, or a substituted or unsubstituted heterocyclic group having 5 to 30 ring atoms.
In the formula (40), Ar¹² and Ar¹³ preferably represent groups different from each other.
In the formula (40), at least one of Ar¹² and Ar¹³ preferably represents a substituent having a substituted or unsubstituted condensed ring group having 10 to 30 ring atoms.

The formula (40) is preferably an anthracene derivative represented by the following formula (41): 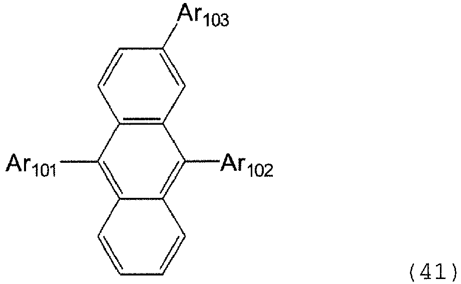 wherein Ar₁₀₁ to Ar₁₀₃ each independently represent a substituted or unsubstituted aryl group having 6 to 30 ring carbon atoms or a substituted or unsubstituted heterocyclic group having 5 to 30 ring atoms.

The anthracene derivative represented by the formula (41) is preferably an anthracene derivative represented by the formula (42), (43) or (44): 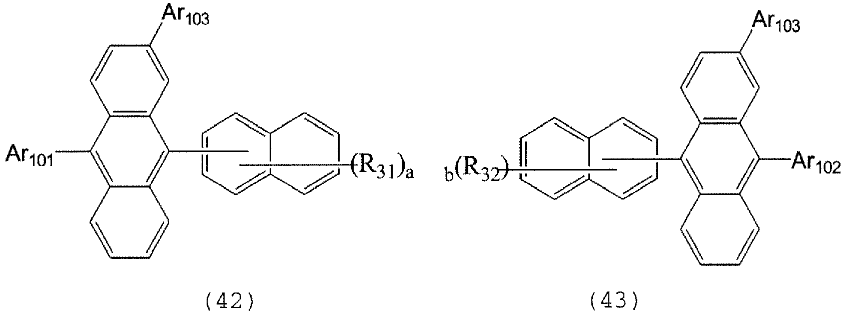 wherein Ar₁₀₁ to Ar₁₀₃ have the same meanings as in the formula (41);
R₃₁ and R₃₂ each independently represent a hydrogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 30 carbon atoms, a substituted or unsubstituted arylsilyl group having 8 to 30 ring carbon atoms, a substituted or unsubstituted aryl group having 6 to 30 ring carbon atoms, or a substituted or unsubstituted heterocyclic group having 5 to 30 ring atoms; and
a and b each independently represent an integer of from 1 to 7.

In the formula (42), when a is 2 or more, plural groups of R₃₁ may be the same as or different from each other, and adjacent groups of R₃₁ may form a ring. The conditions are also applied to b and R₃₂ in the formula (43).

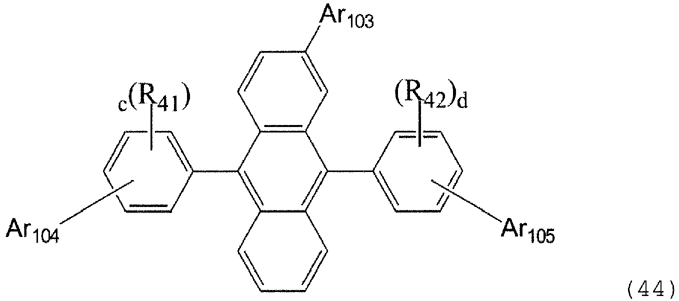

wherein Ar₁₀₃ to Ar₁₀₅ each independently represent a substituted or unsubstituted aryl group having 6 to 30 ring carbon atoms, or a substituted or unsubstituted heterocyclic group having 5 to 30 ring atoms;
R₄₁ and R₄₂ each independently represent a hydrogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 30 carbon atoms, a substituted or unsubstituted arylsilyl group having 8 to 30 ring carbon atoms, a substituted or unsubstituted aryl group having 6 to 30 ring carbon atoms, or a substituted or unsubstituted heterocyclic group having 5 to 30 ring atoms; and
c and d each independently represent an integer of from 1 to 4.

In the formula (44), when c is 2 or more, plural groups of R₄₁ may be the same as or different from each other, and adjacent groups of R₄₁ may form a ring. The conditions are also applied to d and R₄₂.
In the formulae (41), (42), (43) and (44), Ar₁₀₃ preferably represents a substituted or unsubstituted phenyl group.

The formula (40) is preferably an anthracene derivative (45).
The anthracene derivative (45) is an anthracene derivative represented by the formula (40), in which any one of R¹⁰¹, R¹⁰², R¹⁰⁷ and R¹⁰⁸ is a substituted or unsubstituted aryl group having 6 to 30 ring carbon atoms or a heterocyclic group having 5 to 30 ring atoms; all R¹⁰¹, R¹⁰², R¹⁰⁷ and R¹⁰⁸ other than R¹⁰¹, R¹⁰², R¹⁰⁷ or R¹⁰⁸ that represents a substituted or unsubstituted aryl group having 6 to 30 ring carbon atoms or a heterocyclic group having 5 to 30 ring atoms are each a hydrogen atom; any one of R¹⁰³ to R¹⁰⁶ is a substituted or unsubstituted aryl group having 6 to 30 ring carbon atoms or a heterocyclic group having 5 to 30 ring atoms; and all R¹⁰³ to R¹⁰⁶ other than R¹⁰³ to R¹⁰⁶ that represent a substituted or unsubstituted aryl group having 6 to 30 ring carbon atoms or a substituted or unsubstituted heterocyclic group having 5 to 30 ring atoms are each a hydrogen atom.

The anthracene derivative (45) is preferably an anthracene derivative represented by the following formula (46): 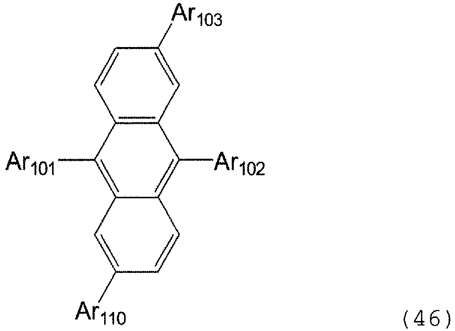 wherein Ar₁₀₁ to Ar₁₀₃ and Ar₁₁₀ each independently represent a substituted or unsubstituted aryl group having 6 to 30 ring carbon atoms or a heterocyclic group having 5 to 30 ring atoms.

The formula (40) is preferably an anthracene derivative represented by the following formula (50): 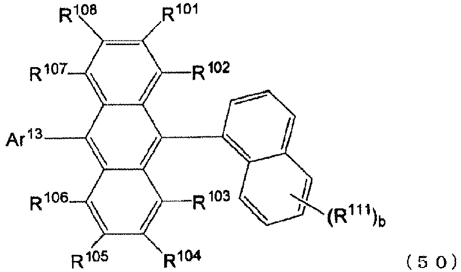 wherein R¹⁰¹ to R¹⁰⁸ and Ar¹³ have the same meanings as in the formula (40);
R¹¹¹ represents a substituted or unsubstituted aryl group having 6 to 30 ring carbon atoms, a substituted or unsubstituted heterocyclic group having 5 to 30 ring atoms, a substituted or unsubstituted alkyl group having 1 to 10 carbon atoms, a substituted or unsubstituted cycloalkyl group having 3 to 10 ring carbon atoms, a substituted or unsubstituted alkoxy group having 1 to 20 carbon atoms, a substituted or unsubstituted aryl- and/or alkylsilyl group, a cyano group or a fluorine atom; and
b represents an integer of from 0 to 7, provided that when b is 2 or more, plural groups of R¹¹¹ may be the same as or different from each other.

In the anthracene derivative of the formula (50), a derivative represented by the following formula (51) is preferred: 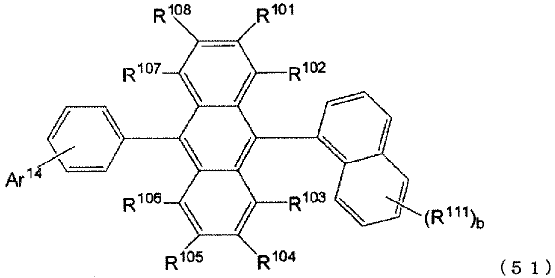 wherein R¹⁰¹ to R¹⁰⁸, R¹¹¹ and b have the same meanings as in the formula (50); and
Ar¹⁴ represents a substituted or unsubstituted aryl group having 6 to 30 ring carbon atoms, a substituted or unsubstituted alkyl group having 1 to 10 carbon atoms, a substituted or unsubstituted cycloalkyl group having 3 to 10 ring carbon atoms, or a substituted or unsubstituted heterocyclic group having 5 to 30 ring atoms.
Ar¹⁴ preferably represents a 9,9-dimethylfluoren-1-yl group, a 9,9-dimethylfluoren-2-yl group, a 9,9-dimethylfluoren-3-yl group, a 9,9-dimethylfluoren-4-yl group, a dibenzofuran-1-yl group, a dibenzofuran-2-yl group, a dibenzofuran-3-yl group or a dibenzofuran-4-yl group.

The anthracene derivative may be, in addition to the formula (50), any one of anthracene derivatives represented by the following formulae (52) to (58): 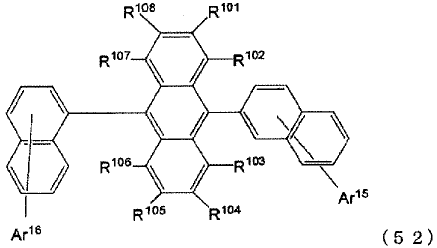 wherein R¹⁰¹ to R¹⁰⁸ have the same meanings as in the formula (50); and
Ar¹⁵ and Ar¹⁶ each represent a hydrogen atom, an aryl group having 6 to 30 ring carbon atoms, or a substituted or unsubstituted heterocyclic group having 5 to 30 ring atoms, provided that Ar¹⁵ may form a ring with the naphthalene ring bonded thereto.

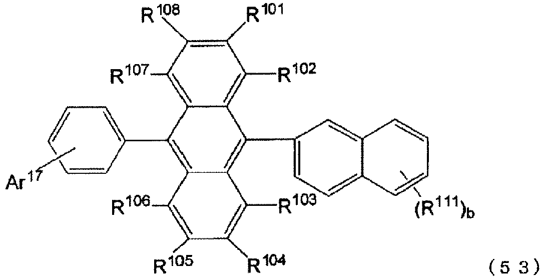

wherein R¹⁰¹ to R¹⁰⁸, R¹¹¹ and b have the same meanings as in the formula (50); and
Ar¹⁷ represents a substituted or unsubstituted aryl group having 6 to 30 ring carbon atoms, a substituted or unsubstituted alkyl group having 1 to 10 carbon atoms, a substituted or unsubstituted cycloalkyl group having 3 to 10 ring carbon atoms, or a substituted or unsubstituted heterocyclic group having 5 to 30 ring atoms.
Ar¹⁷ preferably represents, for example, a dibenzofuran-1-yl group, a dibenzofuran-2-yl group, a dibenzofuran-3-yl group or a dibenzofuran-4-yl group.

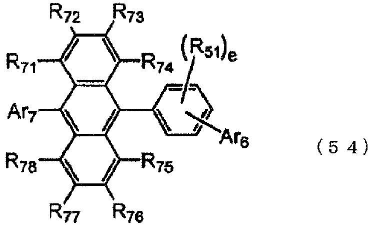

wherein R₇₁ to R₇₈ each independently represent a hydrogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 30 carbon atoms, or a substituted or unsubstituted arylsilyl group having 8 to 30 ring carbon atoms;
R₅₁ represents a hydrogen atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 30 carbon atoms, a substituted or unsubstituted arylsilyl group having 8 to 30 ring carbon atoms, a substituted or unsubstituted aryl group having 6 to 30 ring carbon atoms, or a substituted or unsubstituted heterocyclic group having 5 to 30 ring atoms;
e represents an integer of from 1 to 4;
Ar₆ represents a substituted or unsubstituted aryl group having 6 to 30 ring carbon atoms or a substituted or unsubstituted heterocyclic group having 5 to 30 ring atoms; and
Ar₇ represents a substituted or unsubstituted condensed aryl group having 10 to 20 ring carbon atoms or a substituted or unsubstituted condensed heterocyclic group having 9 to 20 ring atoms.

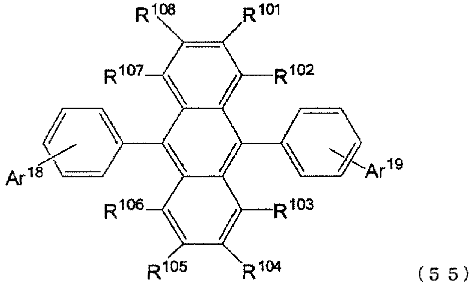

wherein R¹⁰¹ to R¹⁰⁸ have the same meanings as in the formula (50);
Ar¹⁸ represents a substituted or unsubstituted aryl group having 6 to 30 ring carbon atoms, a substituted or unsubstituted alkyl group having 1 to 10 carbon atoms, a substituted or unsubstituted cycloalkyl group having 3 to 10 ring carbon atoms, or a substituted or unsubstituted heterocyclic group having 5 to 50 ring atoms, provided that Ar¹⁸ may form a ring with the benzene ring bonded thereto; and
Ar¹⁹ represents a substituted or unsubstituted aryl group having 6 to 30 ring carbon atoms, a substituted or unsubstituted alkyl group having 1 to 10 carbon atoms, a substituted or unsubstituted cycloalkyl group having 3 to 10 ring carbon atoms, or a substituted or unsubstituted heterocyclic group having 5 to 30 ring atoms.

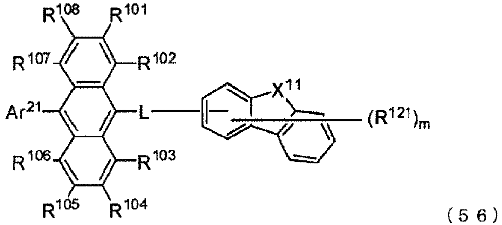

wherein R¹⁰¹ to R¹⁰⁸ have the same meanings as in the formula (50);
L represents a single bond, a substituted or unsubstituted arylene group having 6 to 50 ring carbon atoms, or a substituted or unsubstituted divalent heterocyclic group having 5 to 50 ring atoms;
Ar²¹ represents a substituted or unsubstituted aryl group having 6 to 30 ring carbon atoms or a substituted or unsubstituted heterocyclic group having 5 to 30 ring atoms;
X¹¹ represents an oxygen atom, a sulfur atom, -NR¹³¹ or -CR¹³²R¹³³, wherein R¹³¹, R¹³² and R¹³³ each represent the similar groups as R¹⁰¹ to R¹⁰⁸;
R¹²¹ represents a substituted or unsubstituted aryl group having 6 to 30 ring carbon atoms, a substituted or unsubstituted heterocyclic group having 5 to 30 ring atoms, a substituted or unsubstituted alkyl group having 1 to 10 carbon atoms, a substituted or unsubstituted cycloalkyl group having 3 to 10 ring carbon atoms, a substituted or unsubstituted alkoxy group having 1 to 20 carbon atoms, a substituted or unsubstituted aryl- and/or alkylsilyl group, a cyano group or a fluorine atom; and
m represents an integer of from 0 to 7, provided that when m is 2 or more, plural groups of R¹²¹ may be the same as or different from each other.

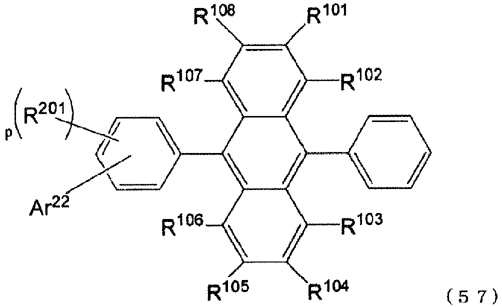

wherein R¹⁰¹ to R¹⁰⁸ have the same meanings as in the formula (50);
Ar²² represents a substituted or unsubstituted aryl group having 6 to 30 ring carbon atoms or a substituted or unsubstituted heterocyclic group having 5 to 30 ring atoms;
R²⁰¹ represents a substituted or unsubstituted aryl group having 6 to 30 ring carbon atoms, a substituted or unsubstituted heterocyclic group having 5 to 30 ring atoms, a substituted or unsubstituted alkyl group having 1 to 10 carbon atoms, a substituted or unsubstituted cycloalkyl group having 3 to 10 ring carbon atoms, a substituted or unsubstituted alkoxy group having 1 to 20 carbon atoms, a substituted or unsubstituted aryl- and/or alkyl silyl group, a cyano group or a fluorine atom; and
q represents an integer of from 0 to 4, provided that when p is 2 or more, plural groups of R²⁰¹ may be the same as or different from each other.

In addition to the above, a compound represented by the following formula (58) may be preferably used as a host material: 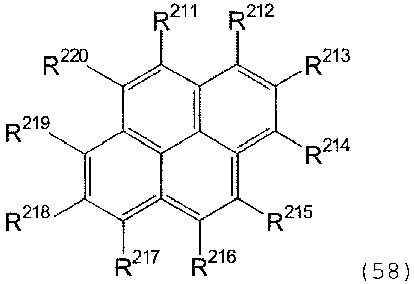 wherein R²¹¹ to R²²⁰ have the same meanings as R¹⁰¹ to R¹⁰⁸ in the formula (50).

In the organic EL device of the present invention, the organic layers including the light emitting layer and the like may be formed by a dry film forming method, such as vacuum vapor deposition, a molecular beam epitaxy method (MBE method), sputtering, plasma, and ion plating, or a coating method of a solution obtained by dissolving in a solvent, such as spin coating, dipping, casting, bar coating, roll coating, flow coating, and ink-jet.
Upon producing an organic EL device with the aminoanthracene derivative of the present invention, in particular, the organic compound layer and the light emitting layer may be formed not only by vapor deposition but also by a wet method.
The thickness of the layers of the organic compound layers is not particularly limited, and is set to a suitable thickness. In general, when the thickness is too small, pinholes and the like are formed to provide a possibility that sufficient light emission luminance is not obtained upon application of an electric field, and when the thickness is too large, a higher voltage is necessarily applied for providing a certain optical output, which deteriorates the efficiency. Thus, the thickness is generally suitably in a range of from 5 nm to 10 µm, and preferably from 10 nm to 0.2 µm.

In the wet film forming method, an organic EL material-containing solution containing the aminoanthracene derivative of the present invention as a material for an organic EL device and a solvent may be used, and the organic EL material-containing solution used is preferably an organic EL material-containing solution containing the aminoanthracene derivative of the present invention and at least one kind selected from the compounds represented by the formulae (50) to (58).
In this case, the organic EL material forming the layers is dissolved or dispersed in a suitable solvent to prepare the organic EL material-containing solution, with which the thin film is formed, and the solvent may be any solvent. Examples of the solvent include a halogenated hydrocarbon solvent, such as dichloromethane, dichloroethane, chloroform, carbon tetrachloride, tetrachloroethane, trichloroethane, chlorobenzene, dichlorobenzene, chlorotoluene and trifluorotoluene, an ether solvent, such as dibutyl ether, tetrahydrofuran, tetrahydropyran, dioxane, anisol and dimethoxyethane, an alcohol solvent, such as methanol, ethanol, propanol, isopropanol, butanol, pentanol, hexanol, cyclohexanol, methyl cellosolve, ethyl cellosolve and ethylene glycol, a ketone solvent, such as acetone, methyl ethyl ketone, diethyl ketone, 2-hexanone, methyl isobutyl ketone, 2-heptanone, 4-heptanone, diisobutyl ketone, acetonylacetone, isophorone, cyclohexanone, methylhexanone and acetophenone, a hydrocarbon solvent, such as benzene, toluene, xylene, ethylbenzene, hexane, cyclohexane, octane, decane and tridecane, an ester solvent, such as ethyl acetate, butyl acetate and amyl acetate, a linear carbonate ester solvent, such as dimethyl carbonate, methyl ethyl carbonate and diethyl carbonate, and a cyclic carbonate ester solvent, such as ethylene carbonate and propylene carbonate. Among these, a hydrocarbon solvent and an ether solvent, such as toluene and dioxane, are preferred. The solvent may be used solely or as a mixture of two or more kinds thereof. The solvent that may be used herein is not limited to these.

Any of the organic compound layers may contain suitable resin and additive for enhancing the film forming property and preventing pinholes or the like from being formed in the film. Examples of the resin that may be used include an insulating resin, such as polystyrene, polycarbonate, polyarylate, polyester, polyamide, polyurethane, polysulfone, polymethyl methacrylate, polymethyl acrylate and cellulose, and copolymers thereof, a photoconductive resin, such as poly-N-vinylcarbazole and polysilane, and an electroconductive resin, such as polyaniline, polythiophene and polypyrrole. Examples of the additive include an antioxidant, an ultraviolet ray absorbent and a plasticizer.
For enhancing the stability of the organic EL device obtained in the present invention to temperature, humidity, atmosphere and the like, a protective layer may be provided on the surface of the device, and the device may be entirely protected with a silicone oil, a resin or the like.
In the organic EL device of the present invention, a layer selected from a chalcogenide layer, a halogenated metal layer and a metal oxide layer is preferably disposed on the surface of at least one of the pair of electrodes.

### Structure of Organic EL Device

The device structure of the organic EL device of the present invention will be described.
Examples of the representative device structure of the organic EL device of the present invention include the following structures.
(1) anode/light emitting layer/cathode
(2) anode/positive hole injection layer/light emitting layer/cathode
(3) anode/light emitting layer/electron transporting layer/cathode
(4) anode/positive hole injection layer/light emitting layer/electron transporting layer/cathode
(5) anode/organic semiconductor layer/light emitting layer/cathode
(6) anode/organic semiconductor layer/electron barrier layer/light emitting layer/cathode
(7) anode/organic semiconductor layer/light emitting layer/adhesion improving layer/cathode
(8) anode/positive hole injection layer/positive hole transporting layer/light emitting layer/electron transporting layer/cathode
(9) anode/insulating layer/light emitting layer/insulating layer/cathode
(10) anode/inorganic semiconductor layer/insulating layer/light emitting layer/insulating layer/cathode
(11) anode/organic semiconductor layer/insulating layer/light emitting layer/insulating layer/cathode
(12) anode/insulating layer/positive hole injection layer/positive hole transporting layer/light emitting layer/insulating layer/cathode
(13) anode/insulating layer/positive hole injection layer/positive hole transporting layer/light emitting layer/electron transporting layer/cathode
Among these, in general, the structure (8) is preferably employed.

The organic EL device of the present invention may be produced on a substrate having light transmissibility. The light transmissible substrate referred herein is preferably a flat and smooth substrate that supports the organic EL device and has a transmittance of 50% or more to light in the visible region with a wavelength of from 400 to 700 nm.
Specific examples thereof include a glass plate and a polymer plate. Examples of the glass plate include soda-lime glass, barium and strontium-containing glass, lead glass, aluminosilicate glass, borosilicate glass, barium borosilicate glass and quartz. Examples of the polymer substrate include polycarbonate, acrylate, polyethylene terephthalate, polyether sulfone and polysulfone.

The anode of the organic EL device of the present invention has a function of injecting positive holes to the positive hole transporting layer or the light emitting layer, and it is effective to have a work function of 4.5 eV or more. Specific examples of the anode material used in the present invention include indium tin oxide (ITO), tin oxide (NESA), gold, silver, platinum and copper. The anode is preferably a material having a small work function for the purpose of injecting electrons to the electron transporting layer or the light emitting layer.
The anode may be produced by forming a thin film of the electrode substance by such a method as a vapor deposition method or a sputtering method.
In the case of extracting light emitted from the light emitting layer through the anode, the anode preferably has a transmittance of 10% or more to the emitted light. The anode preferably has a sheet resistance of several hundred Ω per square or less. While depending on the material of the anode, the thickness of the anode is generally selected from a range of from 10 nm to 1 µm, and preferably from 10 to 200 nm.

The light emitting layer of the organic EL device has the following functions.
(1) Injection function capable of, upon application of an electric field, injecting positive holes from the anode or the positive hole injection layer, and injecting electrons from the cathode or the electron transporting layer.
(2) Transporting function capable of transporting the charge (electrons and positive holes) thus injected with the force of the electric field.
(3) Light emitting function of capable of providing a field for recombination of electrons and positive holes, and deriving the same to light emission.
There may be a difference between the injectability of positive holes and the injectability of electrons, and there may be a difference in transporting capability expressed by mobility between positive holes and electrons, but it is preferred to migrate any one of the charges.

As the method for forming the light emitting layer, any known method may be employed, such as a vapor deposition method, a spin coating method and a Langmuir-Blodgett method. The light emitting layer is particularly preferably a molecular deposition film.
The molecular deposition film referred herein means a film formed by depositing from the material compound in a gas phase or a film formed by solidifying from the material compound in a solution state or a liquid state, and in general, the molecular deposition film can be distinguished from a thin film formed by a Langmuir-Blodgett method (molecular accumulated film) by the differences in aggregated structure and higher order structure, and the functional difference derived therefrom.
The light emitting layer may also be formed in such a manner that the material compound is dissolved in a solvent along with a binder, such as a resin, to form a solution, which is then formed into a thin film by a spin coating method or the like.
In the case where the aminoanthracene derivative of the present invention is used in a light emitting layer, the aminoanthracene derivative of the present invention may be used as either the dopant material or the host material, and is particularly preferably used as a dopant material.
In the present invention, the light emitting layer may contain, depending on necessity, other known light emitting materials than the light emitting material containing the aminoanthracene derivative of the present invention and the condensed ring-containing compound in such a range that does not impair the objects of the present invention, and a light emitting layer containing the other known light emitting materials may be laminated on the light emitting layer containing the light emitting material of the present invention.
The light emitting layer preferably has a thickness of from 5 to 50 nm, more preferably from 7 to 50 nm, and most preferably from 10 to 50 nm. When the thickness is less than 5 nm, the light emitting layer may be difficult to be formed to make the chromaticity unadjustable, and when it exceeds 50 nm, the driving voltage may be increased.

The positive hole injection and transporting layers facilitate injection of positive holes and transport to the light emitting region, and have a large positive hole mobility and a small ionization energy of generally 5.5 eV or less. The positive hole injection and transporting layers are preferably a material that transports positive holes to the light emitting layer with a lower electric field intensity, and preferably has a positive hole mobility of, for example, at least 10⁻⁴ cm²/V·sec upon application of an electric field of from 104 to 106 V/cm.
Specific examples thereof include an arylamine derivative, an arylcarbazole derivative, a triazole derivative, an oxadiazole derivative, an imidazole derivative, a polyarylalkane derivative, a pyrazoline derivative, a pyrazolone derivative, a phenylenediamine derivative, an amino-substituted chalcone derivative, an oxazole derivative, a styrylanthracene derivative, a fluorenone derivative, a hydrazone derivative, a stilbene derivative, a silazane derivative, a polysilane copolymer and an aniline copolymer.

Examples thereof also include 4,4'-bis(N-(1-naphthyl)-N-phenylamino)biphenyl (which may be hereinafter abbreviated as NPD) having two condensed aromatic rings in the molecule, and 4,4',4"-tris(N-(40-methylphenyl)-N-phenylamino)triphenylamine (which may be hereinafter abbreviated as MTDATA) having three triphenylamine units bonded to a starburst form.
In addition to the aromatic dimethylidyne compounds described above for the material of the light emitting layer, an inorganic compound, such as p-type Si and p-type SiC, may be used as the material of the positive hole injection layer.
The positive hole injection and transporting layers may be formed by making the aforementioned compound into a thin film by a known method, such as a vacuum vapor deposition method, a spin coating method, a casting method and a Langmuir-Blodgett method. The thickness of the positive hole injection and transporting layers is not particularly limited, and is generally from 5 nm to 5 µm.

The organic electroluminescent device of the present invention, which contains a pair of electrodes, and therebetween one or plural organic thin film layers containing at least a light emitting layer, is preferably an organic electroluminescent device that has the light emitting layer containing at least one kind of the aminoanthracene derivative of the present invention and at least one kind of the compound represented by the formula (50), and a positive hole transporting layer and/or a positive hole injection layer containing at least one kind selected from an arylamine derivative and an arylcarbazole derivative.

The electron transporting layer facilitates injection of electrons to the light emitting layer and has a large electron mobility, and the electron transporting layer may be constituted by one layer or plural layers. The adhesion improving layer is the electron transporting layer that exhibits good adhesion particularly to the cathode. Preferred examples of the material used in the electron transporting layer include metal complexes of 8-hydroxyquinoline and a derivative thereof.
Specific examples of the metal complexes of 8-hydroxyquinoline and a derivative thereof include a metal chelate oxinoid compound containing a chelate of oxine (which is generally referred to as 8-quinolinol or 8-hydroxyquinoline).
Alq described for the light emitting material may be used as the electron transporting layer.

Examples of the oxadiazole derivative include electron transfer compounds represented by the following formulae:

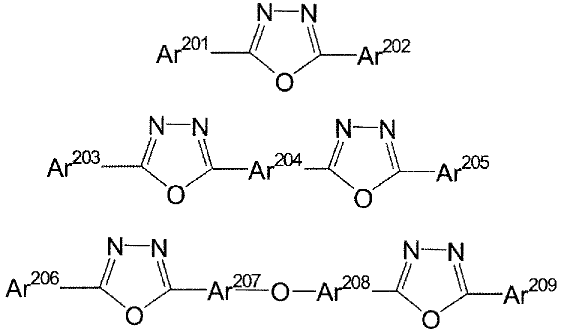

wherein Ar²⁰¹, Ar²⁰², Ar²⁰³ , Ar²⁰⁵, Ar²⁰⁶ and Ar²⁰⁹ each represent a substituted or unsubstituted aryl group and may be the same as or different from each other; and Ar²⁰⁴ , Ar²⁰⁷ and Ar²⁰⁸ each represent a substituted or unsubstituted arylene group and may be the same as or different from each other.
The electron transfer compound preferably has thin film forming property.

Compounds represented by the following formulae may also be used as the electron transporting material, i.e., nitrogen-containing heterocyclic derivatives represented by the following formulae:

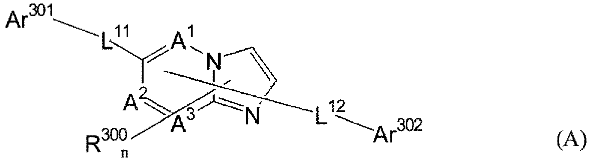

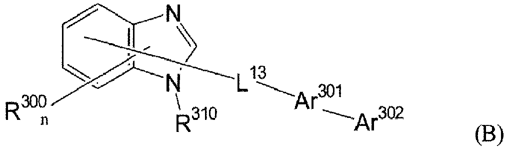

wherein in the formulae (A) and (B), A¹ to A³ each independently represent a nitrogen atom or a carbon atom;
Ar³⁰¹ represents a substituted or unsubstituted aryl group having 6 to 60 ring carbon atoms or a substituted or unsubstituted heterocyclic group having 5 to 60 ring atoms; Ar³⁰² represents a hydrogen atom, a substituted or unsubstituted aryl group having 6 to 60 ring carbon atoms, a substituted or unsubstituted heterocyclic group having 5 to 60 ring atoms, a substituted or unsubstituted alkyl group having 1 to 20 carbon atoms, a substituted or unsubstituted alkoxy group having 1 to 20 carbon atoms, or divalent groups thereof, provided that any one of Ar³⁰¹ and Ar³⁰² represents a substituted or unsubstituted condensed ring group having 10 to 60 ring carbon atoms or a substituted or unsubstituted monohetero condensed ring group having 5 to 60 ring atoms;
L¹¹, L¹² and L¹³ each independently represent a single bond, a substituted or unsubstituted arylene group having 6 to 60 ring carbon atoms, a substituted or unsubstituted heteroarylene group having 5 to 60 ring atoms, or a substituted or unsubstituted fluorenylene group;
R³⁰⁰ represents a hydrogen atom, a substituted or unsubstituted aryl group having 6 to 60 ring carbon atoms, a substituted or unsubstituted heterocyclic group having 5 to 60 ring atoms, a substituted or unsubstituted alkyl group having 1 to 20 carbon atoms, or a substituted or unsubstituted alkoxy group having 1 to 20 carbon atoms; n represents an integer of from 0 to 5, provided that when n is 2 or more, plural groups of R³⁰⁰ may be the same as or different from each other, and plural groups of R³⁰⁰ adjacent to each other may be bonded to form a carbocyclic aliphatic ring or a carbocyclic aromatic ring;
R³⁰¹ represents a hydrogen atom, a substituted or unsubstituted aryl group having 6 to 60 ring carbon atoms, a substituted or unsubstituted heteroaryl group having 3 to 60 ring atoms, a substituted or unsubstituted alkyl group having 1 to 20 carbon atoms, a substituted or unsubstituted alkoxy group having 1 to 20 carbon atoms, or -L¹¹-Ar³⁰¹-Ar³⁰², and

a nitrogen-containing heterocyclic derivative represented by the following formula:

HAr- L¹⁴ - Ar⁴⁰¹ - Ar⁴⁰² (C)

wherein HAr represents a nitrogen-containing heterocyclic ring, which may have a substituent, L¹⁴ represents a single bond, an arylene group having 6 to 60 ring atoms, which may have a substituent, a heteroarylene group having 5 to 60 ring atoms, which may have a substituent, or a fluorenylene group, which may have a substituent; Ar⁴⁰¹ represents an aryl group having 6 to 60 ring carbon atoms, which may have a substituent; and Ar⁴⁰² represents an aryl group having 6 to 60 ring carbon atoms, which may have a substituent, or a heterocyclic group having 5 to 60 ring atoms, which may have a substituent.

The organic electroluminescent device of the present invention, which contains a pair of electrodes, and therebetween one or plural organic thin film layers containing at least a light emitting layer, is preferably an organic electroluminescent device that has the light emitting layer containing at least one kind of the aminoanthracene derivative of the present invention and at least one kind of the compound represented by the formula (50), and an electron transporting layer containing at least one kind of the compounds represented by the formulae (A), (B) and (C).
The organic electroluminescent device, which contains a pair of electrodes, and therebetween one or plural organic thin film layers containing at least a light emitting layer, is preferably an organic electroluminescent device that has the light emitting layer containing at least one kind of the aminoanthracene derivative of the present invention and at least one kind of the compound represented by the formula (50), a positive hole transporting layer and/or a positive hole injection layer containing at least one kind selected from an arylamine derivative and an arylcarbazole derivative, and an electron transporting layer containing at least one kind of the compounds represented by the formulae (A), (B) and (C).

In addition, nitrogen-containing heterocyclic derivatives represented by the following formulae (III) and (IV) may also be used in the organic EL device of the present invention:

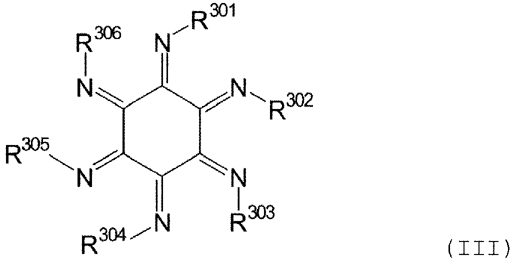

wherein in the formula (III), R³⁰¹, R³⁰², R³⁰³, R³⁰⁴, R³⁰⁵ and R³⁰⁶ each represent a substituted or unsubstituted alkyl group, a substituted or unsubstituted aromatic group, a substituted or unsubstituted aralkyl group or a substituted or unsubstituted heterocyclic group, provided that R³⁰¹, R³⁰², R³⁰³, R³⁰⁴, R³⁰⁵ and R³⁰⁶ may be the same as or different from each other, and R³⁰¹ and R³⁰², R³⁰³ and R³⁰⁴, and R³⁰⁵ and R³⁰⁶, or R³⁰¹ and R³⁰⁶, R³⁰² and R³⁰³, and R³⁰⁴ and R³⁰⁵ may each form a condensed ring.

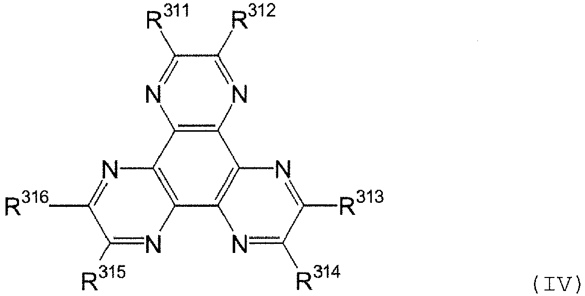

wherein in the formula (IV), R³¹¹ to R³¹⁶ each represent a substituent, and preferably an electron attracting group, such as a cyano group, a nitro group, a sulfonyl group, a carbonyl group, a trifluoromethyl group and a halogen atom.
The organic electroluminescent device of the present invention, which contains a pair of electrodes, and therebetween one or plural organic thin film layers containing at least a light emitting layer, is preferably an organic electroluminescent device that has the light emitting layer containing at least one kind of the aminoanthracene derivative of the present invention and at least one kind of the compound represented by the formula (50), a positive hole transporting layer and/or a positive hole injection layer containing at least one kind selected from an arylamine derivative and an arylcarbazole derivative, an electron transporting layer containing at least one kind of the compounds represented by the formulae (A), (B) and (C), and an organic compound layer containing at least one kind of the compound represented by the formula (IV).

An inorganic compound, such as p-type Si and p-type SiC, may be used as the material of the positive hole injection layer.
One of preferred embodiments of the organic EL device of the present invention is a device that contains a reduction-causing dopant in an interfacial region between the region where electrons are transported or the cathode and the organic layer. The reduction-causing dopant referred therein is defined as a substance that is capable of reducing an electron transporting compound. Accordingly, various substances may be used that have certain reducing property, and at least one substance selected from the group consisting of an alkali metal (such as Li, Na, K, Rb and Cs), an alkaline earth metal (such as Ca, Sr and Ba), a rare earth metal, an oxide of an alkali metal, a halogenide of an alkali metal, an oxide of an alkaline earth metal, a halogenide of an alkaline earth metal, an oxide of a rare earth metal, a halogenide of a rare earth metal, an organic complex of an alkali metal, an organic complex of an alkaline earth metal, and an organic complex of a rare earth metal may be preferably used. A substance having a work function of 2.9 eV or less is particularly preferred. The alkali metals have particularly high reducing capability, and the addition thereof in a relatively small amount to the electron injection region enhances the light emission luminance and prolongs the lifetime. Preferred examples of the reduction-causing dopant having a work function of 2.9 eV or less include a combination of two or more of the alkali metals.

In the present invention, an electron transporting layer constituted by an insulating material or a semiconductor may be provided between the cathode and the organic layer. In this case, leakage of electric current is effectively prevented to enhance the electron injecting property. The insulating material used is preferably at least one metal compound selected from the group consisting of an alkali metal chalcogenide (such as Li₂O, K₂O, Na₂S, Na₂Se and Na₂O), an alkaline earth metal chalcogenide (such as CaO, BaO, SrO, BeO, BaS and CaSe), a halogenide of an alkali metal (such as LiF, NaF, KF, LiCl, KCl and NaCl) and a halogenide of an alkaline earth metal (such as CaF₂, BaF₂, SrF₂, MgF₂ and BeF₂).

Examples of the semiconductor constituting the electron transporting layer include a sole material and a combination of two or more kinds of oxides, nitrides and oxynitrides of containing at least one element of Ba, Ca, Sr, Yb, Al, Ga, In, Li, Na, Cd, Mg, Si, Ta, Sb and Zn. The inorganic compound constituting the electron transporting layer is preferably an insulating thin film of microcrystals or an amorphous material. The electron transporting layer formed of the insulating thin film can be formed into a more uniform thin film, thereby decreasing image defects, such as dark sports. Examples of the inorganic material include the alkali metal chalcogenide, the alkaline earth metal chalcogenide, the halogenide of an alkali metal and the halogenide of an alkaline earth metal, described above.

The cathode used may contain, as an electrode substance, a metal, an alloy and an electroconductive compound that have a small work function (4 eV or less), and a mixture thereof, for injecting electrons to the electron transporting layer or the light emitting layer. Specific examples of the electrode substance include sodium, a sodium-potassium alloy, magnesium, lithium, a magnesium-silver alloy, aluminum-aluminum oxide, an aluminum-lithium alloy, indium and a rare earth metal.
The cathode may be produced by forming a thin film of the electrode substance by such a method as vapor deposition and sputtering.
In the case of extracting light emitted from the light emitting layer through the cathode, the cathode preferably has a transmittance of 10% or more to the emitted light.
The cathode preferably has a sheet resistance of several hundred Ω per square or less. While depending on the material of the cathode, the thickness of the cathode is generally from 10 nm to 1 µm, and preferably from 50 to 200 nm.

In an organic EL device, an electric field is applied to an ultrathin film, and thus image defects are liable to occur due to leakage or short circuit. For preventing these, an insulating thin film layer is preferably inserted between the pair of electrodes.
Examples of the material used in the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide.
Mixtures and laminated structures thereof may be used.

The organic EL device may be produced by forming the anode, the light emitting layer, the positive hole injection layer depending on necessity, and the electron transporting layer depending on necessity, and further forming the cathode, by using the materials and forming methods described above. The organic EL device may be produced by forming in the reverse order, i.e., from the cathode to the anode.
An example of production of the organic EL device having the structure of anode/positive hole injection layer/light emitting layer/electron transporting layer/cathode provided on a transparent substrate in this order will be described below.
On a suitable transparent substrate, a thin film formed of an anode material is formed to a thickness of 1 µm or less, and preferably in a range of from 10 to 200 nm, by such a method as vapor deposition or sputtering, thereby producing an anode. A positive hole injection layer is then formed on the anode. The positive hole injection layer may be formed by such a method as a vacuum vapor deposition method, a spin coating method, a casting method or a Langmuir-Blodgett method, as described above, and is preferably formed by a vacuum vapor deposition method from the standpoint that a uniform film is obtained, and pinholes are difficult to be formed. In the case where the positive hole injection layer is formed by a vacuum vapor deposition method, the vapor deposition conditions are generally preferably selected from ranges of a temperature of the evaporation source of from 50 to 450ºC, a degree of vacuum of from 10⁻⁷ to 10⁻³ Torr, a vapor deposition rate of from 0.01 to 50 nm/sec, a substrate temperature of from -50 to 300ºC, and a film thickness of from 5 nm to 5 µm, while they vary depending on the crystalline structure and the recombination structure of the target positive hole injection layer.

The light emitting layer may be then formed on the positive hole injection layer by forming a desired organic light emitting material into a thin film by such a method as a vacuum vapor deposition method, a sputtering method, a spin coating method or a casting method, and is preferably formed by a vacuum vapor deposition method from the standpoint that a uniform film is obtained, and pinholes are difficult to be formed. In the case where the light emitting layer is formed by a vacuum vapor deposition method, the vapor deposition conditions may be generally selected from the similar ranges as the positive hole injection layer, while the vapor deposition conditions vary depending on the compound used.
The electron transporting layer is then provided on the light emitting layer. The electron transporting layer is preferably formed by a vacuum vapor deposition method as similar to the positive hole injection layer and the light emitting layer owing to the necessity of providing a uniform film. The vapor deposition conditions may be selected from the similar ranges as the positive hole injection layer and the light emitting layer.
The compound of the present invention may be co-vapor-deposited with other materials in the case where a vacuum vapor deposition method is used, or may be mixed with other materials to be contained in the case where a spin coating method is used.
The cathode is laminated finally, thereby providing the organic EL device.
The cathode may be constituted by a metal and may be formed by a vapor deposition method or a sputtering method, and preferably by a vapor deposition method for protecting the organic material layers thereunder from damages upon forming the film.
In the production of the organic EL device described above, the production of from the anode to the cathode is preferably performed continuously within single vacuuming operation.

The thicknesses of the organic layers of the organic EL device of the present invention are not particularly limited and are generally preferably in a range of from several nanometer to 1 µm since too small a thickness may cause defects, such as pinholes, and too large a thickness may require a high application voltage, which deteriorates the efficiency.
In the case where a direct current voltage is applied to the organic EL device, light emission is observed upon application of a voltage of from 5 to 40 V with the anode for the positive pole and the cathode for the negative pole. Upon application of a voltage with the reverse polarity, no electric current flows, and no light emission occurs. Upon application of an alternating current voltage, uniform light emission is observed only when the anode becomes the positive pole and the cathode becomes the negative pole. The alternating current to be applied may have an arbitrary waveform.

The organic EL device of the present invention may be applied to a planar light emission material for a flat panel display of a wall-mounted television set, a duplicator, a printer, a backlight of a liquid crystal display device, a light source for a measuring instrument gauge, a display panel, a marker light, and the like. The material of the present invention may be used not only in an organic EL device, but also in such fields as an electrophotographic photoreceptor, a photoelectric conversion device, a solar cell and an image sensor.

### Example

The present invention will be described in more detail with reference to examples below, but the present invention is not limited to the examples unless the gist thereof is deviated.

### Synthesis Example 1 (Synthesis of Compound D16)

A compound was synthesized according to the following scheme.

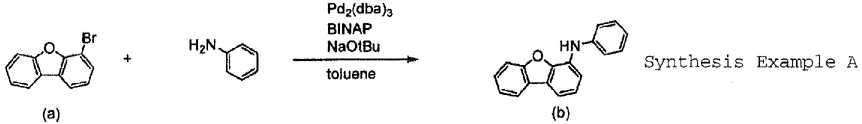

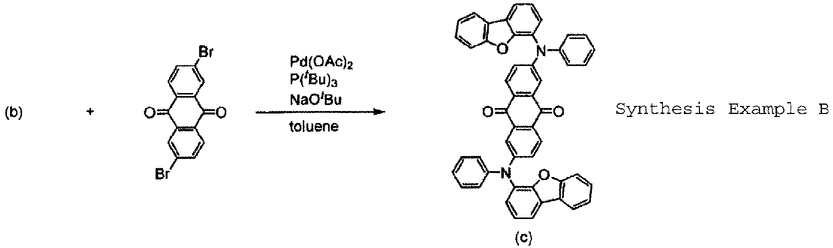

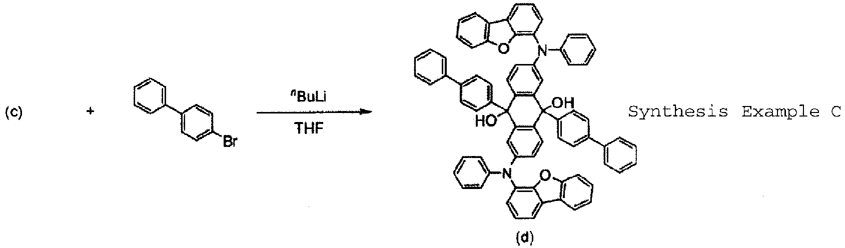

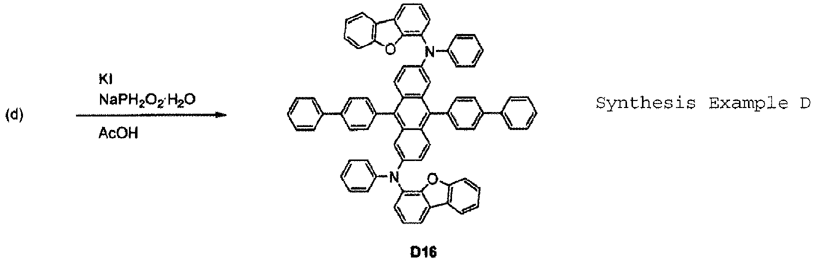

### (1) Synthesis of Intermediate (b) (Synthesis Example A)

11.7 g of the intermediate (a), 10.7 mL of aniline, 0.63 g of tris(dibenzylideneacetone) dipalladium(0) (Pd₂(dba)₃), 0.87 g of 2,2'-bis(diphenylphosphino)-1,1'-binaphthyl (BINAP), 9.1 g of sodium tert-butoxide and 131 mL of dehydrated toluene were placed in a 300-mL recovery flask under an argon stream, and reacted at 85ºC for 6 hours.
After cooling, the reaction solution was filtered with Celite, the resulting crude product was purified by silica gel chromatography (n-hexane/methylene chloride (3/1)), and the resulting solid was dried under reduced pressure, thereby providing 10.0 g of a white solid, which was identified as the intermediate (b) by analysis with FD-MS (field desorption mass spectrum).

### (2) Synthesis of Intermediate (c) (Synthesis Example B)

16 g of the intermediate (b), 10 g of 2,6-dibromoanthraquinone, 5.5 g of sodium tert-butoxide, 310 mg of palladium(II) acetate (Pd(OAc)₂), 280 mg of tri-tert-butylphosphine and 160 mL of dehydrated toluene were placed in a 500-mL recovery flask under an argon stream, and reacted under refluxing for 7 hours.
After cooling, methanol was added to the reaction solution, the crude product obtained by filtration was purified by silica gel chromatography (toluene), the resulting solid was recrystallized from toluene, and the resulting solid was dried under reduced pressure, thereby providing 13 g of a dark red solid, which was identified as the intermediate (c) by analysis with FD-MS.

### (3) Synthesis of Intermediate (d) (Synthesis Example C)

4.0 g of the intermediate (c) and 100 mL of dehydrated THF were placed in a 300-mL recovery flask under an argon stream, and after cooling to -65ºC, 18 mL (1.57 M) of a hexane solution of n-butyl lithium was added thereto, followed by reacting for 1 hour. 6.5 g of 4-bromodiphenyl was added thereto, followed by reacting for 2 hours, at the same temperature, and then the temperature was gradually increased, followed by reacting at room temperature for 3 hours.
An ammonium chloride saturated aqueous solution was added to the reaction solution, which was then separated, and the organic layer was washed with a saturated sodium chloride aqueous solution, dried over magnesium sulfate, and concentrated to provide a crude product. The crude product was purified by silica gel chromatography (ethyl acetate/hexane) and dried under reduced pressure, thereby providing 5.9 g of a brown solid, which was identified as the intermediate (d) by analysis with FD-MS.

### (4) Synthesis of Compound D16 (Synthesis Example D)

5.9 g of the intermediate (d), 2.4 g of potassium iodide, 760 mg of sodium phosphinate monohydrate and 100 mL of acetic acid were placed in a 300-mL recovery flask and were reacted under reflux of acetic acid for 6.5 hours.
After cooling, methanol was added to the reaction solution, the crude product obtained by filtration was purified by silica gel chromatography (ethyl acetate/toluene/hexane), the resulting solid was washed with dioxane and toluene, and the resulting solid was dried under reduced pressure, thereby providing 1.1 g of a yellowish white solid, which was identified as Compound D16 by analysis with FD-MS.

### Synthesis Example 2 (Synthesis of Compound D26)

A compound was synthesized according to the following scheme. 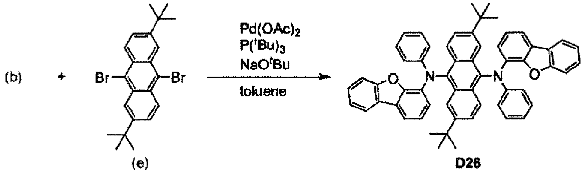 A compound was synthesized in the same manner as in the synthesis of the intermediate (c) in Synthesis Example 1 except that the intermediate (e) was used instead of 2,6-dibromoanthraquinone. The product was identified as Compound D26 by analysis with FD-MS.

### Synthesis Example 3 (Synthesis of Compound D41)

A compound was synthesized according to the following scheme.

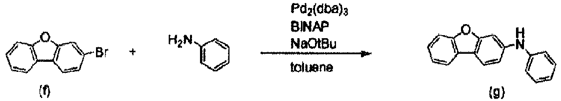

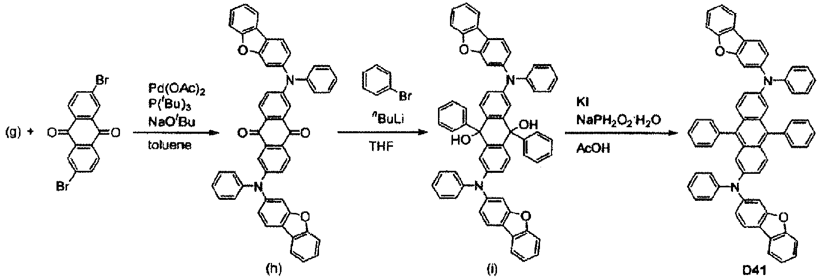

A compound was synthesized in the same manner as in Synthesis Example A in Synthesis Example 1 except that the intermediate (f) was used instead of the intermediate (a). The product was identified as the intermediate (g) by analysis with FD-MS.
A compound was synthesized in the same manner as in Synthesis Example B except that the intermediate (g) was used instead of the intermediate (b). The product was identified as the intermediate (h) by analysis with FD-MS.
A compound was synthesized in the same manner as in Synthesis Example C except that the intermediate (h) was used instead of the intermediate (c), and bromobenzene was used instead of 4-bromodiphenyl. The product was identified as the intermediate (i) by analysis with FD-MS.
A compound was synthesized in the same manner as in Synthesis Example D except that the intermediate (i) was used instead of the intermediate (d). The product was identified as Compound D41 by analysis with FD-MS.

### Synthesis Example 4 (Synthesis of Compound D71)

A compound was synthesized according to the following scheme. 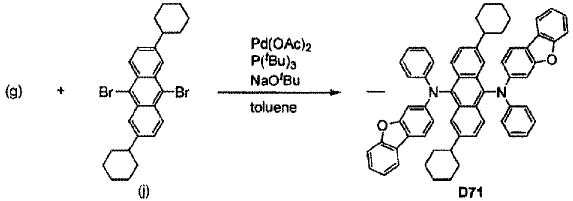 A compound was synthesized in the same manner as in Synthesis Example 2 except that the intermediate (g) was used instead of the intermediate (b), and the intermediate (j) was used instead of the intermediate (e). The product was identified as Compound D71 by analysis with FD-MS.

### Synthesis Example 5 (Synthesis of Compound D81)

A compound was synthesized according to the following scheme.

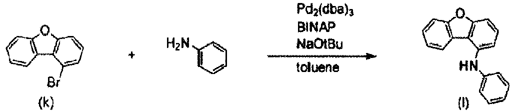

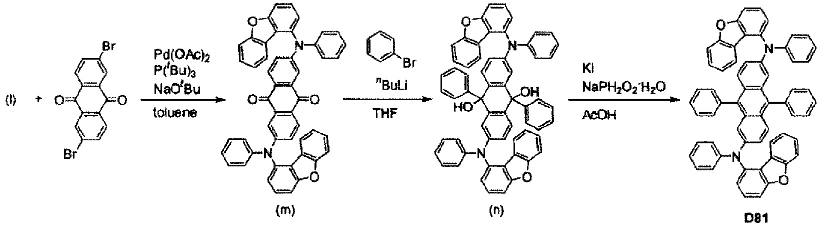

A compound was synthesized in the same manner as in Synthesis Example A in Synthesis Example 1 except that the intermediate (k) was used instead of the intermediate (a). The product was identified as the intermediate (1) by analysis with FD-MS.
A compound was synthesized in the same manner as in Synthesis Example B except that the intermediate (1) was used instead of the intermediate (b). The product was identified as the intermediate (m) by analysis with FD-MS.
A compound was synthesized in the same manner as in Synthesis Example C except that the intermediate (m) was used instead of the intermediate (c), and bromobenzene was used instead of 4-bromodiphenyl. The product was identified as the intermediate (n) by analysis with FD-MS.
A compound was synthesized in the same manner as in Synthesis Example D except that the intermediate (n) was used instead of the intermediate (d). The product was identified as Compound D81 by analysis with FD-MS.

### Synthesis Example 6 (Synthesis of Compound D116)

A compound was synthesized according to the following scheme.

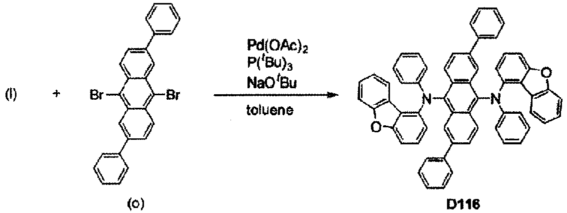

A compound was synthesized in the same manner as in Synthesis Example 2 except that the intermediate (1) was used instead of the intermediate (b), and the intermediate (o) was used instead of the intermediate (e). The product was identified as Compound D116 by analysis with FD-MS.

### Example 1

A transparent electrode formed of indium tin oxide having a thickness of 130 nm was provided on a glass substrate having a dimension of 25 x 75 x 0.7 mm. The glass substrate was cleaned by irradiating with an ultraviolet ray and ozone, and the substrate was placed in a vacuum vapor deposition equipment.
N',N"-Bis(4-(diphenylamino)phenyl)-N',N"-diphenylbiphenyl-4,4'-diamine was vapor-deposited to a thickness of 60 nm as a positive hole injection layer, and N,N,N',N'-tetrakis(4-biphenyl)-4,4'-benzidine was vapor-deposited thereon to a thickness of 20 nm as a positive hole transporting layer. 9-(2-Naphthyl)-10-(4-(1-naphthyl)phenyl)anthracene as a host material and Compound D16 as a doping material were then simultaneously vapor-deposited at a weight ratio of 40/2, thereby forming a light emitting layer having a thickness of 40 nm.
Subsequently, tris(8-hydroxyquinolinato)aluminum was vapor-deposited to a thickness of 20 nm as an electron transporting layer. Lithium fluoride was then vapor-deposited to a thickness of 1 nm, and then aluminum was vapor-deposited to a thickness of 150 nm. The aluminum/lithium fluoride layer functioned as a cathode. Thus, an organic EL device was completed.
The resulting device was subjected to an operation test, and thus blue light emission (light emission maximum wavelength: 509 nm) with a luminous efficiency of 19.3 cd/A and a light emission luminance of 1,930 cd/m² was obtained at a voltage of 5.9 V and an electric current density of 10 mA/cm². A continuous operation test with a direct current was performed with an initial luminance of 3,000 cd/m², and thus the half decay lifetime was 4,000 hours.

### Example 2

An organic EL device was produced in the same manner as in Example 1 except that Compound D26 was used instead of Compound D16 as a doping material.
The resulting device was subjected to an operation test, and thus blue light emission (light emission maximum wavelength: 499 nm) with a luminous efficiency of 18.0 cd/A and a light emission luminance of 1,800 cd/m² was obtained at a voltage of 6.0 V and an electric current density of 10 mA/cm². A continuous operation test with a direct current was performed with an initial luminance of 3,000 cd/m², and thus the half decay lifetime was 3,500 hours.

### Comparative Example 1

An organic EL device was produced in the same manner as in Example 1 except that Comparative Compound 1 was used instead of Compound D16 as a doping material.
The resulting device was subjected to an operation test, and thus blue light emission (light emission maximum wavelength: 530 nm) with a luminous efficiency of 7.9 cd/A and a light emission luminance of 790 cd/m² was obtained at a voltage of 6.8 V and an electric current density of 10 mA/cm². A continuous operation test with a direct current was performed with an initial luminance of 3,000 cd/m², and thus the half decay lifetime was 1,300 hours.

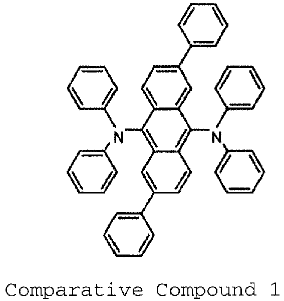

### Industrial Applicability

As described above in detail, the organic EL device using the aminoanthracene derivative of the present invention has a high luminous efficiency and is hard to be deteriorated to have a long lifetime. Accordingly, it is useful as a bright green light emission material of a planar light emission material for a wall-mounted television set and a display device.

## Claims

1. An aminoanthracene derivative represented by the following formula (1): 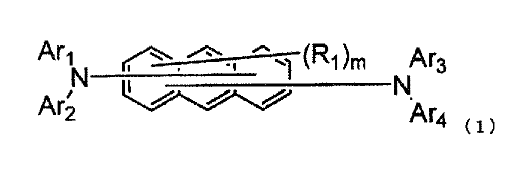 wherein R₁ represents a hydrogen atom, a halogen atom, a substituted or unsubstituted aryl group having 6 to 50 ring carbon atoms, a substituted or unsubstituted a heterocyclic group having 5 to 50 ring atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 7 to 50 ring carbon atoms, a substituted or unsubstituted aryloxy group having 6 to 50 ring carbon atoms, a substituted or unsubstituted arylthio group having 6 to 50 ring carbon atoms, a substituted or unsubstituted alkoxycarbonyl group having 2 to 50 carbon atoms, a substituted or unsubstituted aryl- and/or alkylsilyl group, a carboxyl group, an amino group, a cyano group, a nitro group or a hydroxyl group; m represents an integer of from 1 to 8, provided that when m is 2 or more, plural groups of R₁ may be the same as or different from each other;
Ar₁ to Ar₄ each independently represent a substituted or unsubstituted aryl group having 6 to 50 ring carbon atoms, a substituted or unsubstituted alkyl group having 1 to 50 carbon atoms, a substituted or unsubstituted cycloalkyl group having 3 to 50 ring carbon atoms, a substituted or unsubstituted aralkyl group having 7 to 50 ring carbon atoms, or a substituted or unsubstituted heterocyclic group having 5 to 50 ring atoms,
provided that one or more of Ar₁ to Ar₄ represents a group represented by the following formula (2), (3) or (4): 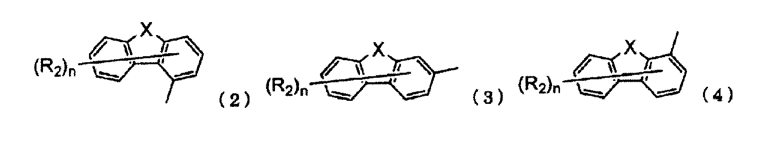 wherein X represents oxygen (O), sulfur (S) or selenium (Se); R₂ represents a hydrogen atom, a substituted or unsubstituted aryl group having 6 to 60 ring carbon atoms, a substituted or unsubstituted alkyl group having 1 to 50 carbon atoms, a substituted or unsubstituted alkenyl group having 2 to 50 carbon atoms, an amino group, a substituted or unsubstituted alkoxy group having 1 to 50 carbon atoms, a substituted or unsubstituted aryloxy group having 6 to 50 ring carbon atoms, a substituted or unsubstituted arylthio group having 6 to 50 ring carbon atoms, a substituted or unsubstituted alkoxycarbonyl group having 1 to 50 carbon atoms, a halogen atom, a cyano group, a nitro group, a hydroxyl group, a substituted or unsubstituted aryl- and/or alkylsilyl group, or a carboxyl group; and n represents an integer of from 1 to 7, provided that when n is 2 or more, plural groups of R₂ may be the same as or different from each other; when there are plural groups of R₂, the groups may be bonded to form a saturated or unsaturated divalent group, which may be substituted; and a case where R₂ is bonded to the 5-membered ring moiety containing X is excluded.

2. The aminoanthracene derivative according to claim 1, wherein the formula (1), Ar₁ and Ar₃ are each a group represented by the formula (2), (3) or (4).

3. The aminoanthracene derivative according to claim 1, wherein Ar₁ and Ar₃ are each a group represented by the formula (4).

4. The aminoanthracene derivative according to claim 1, wherein in the formulae (2), (3) and (4), X represents an oxygen atom.

5. The aminoanthracene derivative according to claim 1, wherein the formula (1) represents the following formula (6): 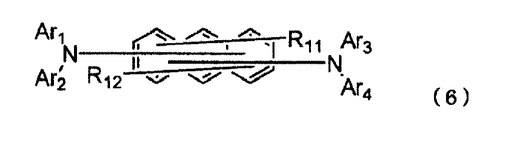 wherein Ar₁ to Ar₄ have the same meanings as above; and R₁₁ and R₁₂ each have the same meaning as R₁.

6. The aminoanthracene derivative according to claim 5, wherein -NAr₁Ar₂ is bonded to the 2-position of the anthracenylene group in the formula (6), and -Nar₃Ar₄ is bonded to the 6-position thereof.

7. The aminoanthracene derivative according to claim 5, wherein -NAr₁Ar₂ is bonded to the 9-position of the anthracenylene group in the formula (6), and -Nar₃Ar₄ is bonded to the 10-position thereof.

8. The aminoanthracene derivative according to claim 1, which is a light emitting material for an organic electroluminescent device.

9. The aminoanthracene derivative according to claim 1, which is a doping material for an organic electroluminescent device.

10. An organic electroluminescent device comprising a cathode and an anode, and therebetween one or plural organic thin film layers containing at least a light emitting layer, the light emitting layer containing the aminoanthracene derivative according to any one of claim 1 to 8 solely or as a component of a mixture.

11. The organic electroluminescent device according to claim 10, wherein the light emitting layer contains the aminoanthracene derivative and an anthracene derivative represented by the following formula (40), and a content of the aminoanthracene derivative is from 0.1 to 20% by weight: 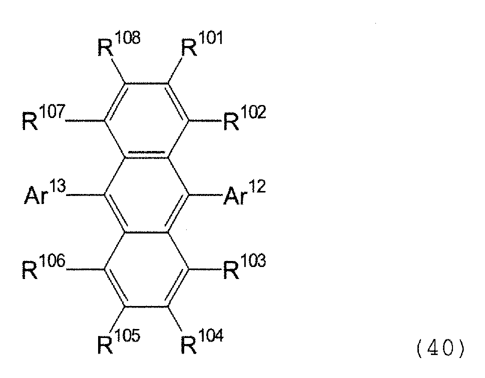 wherein in the formula (40), R¹⁰¹ to R¹⁰⁸ each represent a hydrogen atom, a fluorine atom, a substituted or unsubstituted alkyl group having 1 to 10 carbon atoms, a substituted or unsubstituted cycloalkyl group having 3 to 10 carbon atoms, a substituted or unsubstituted alkylsilyl group having 3 to 30 carbon atoms, a substituted or unsubstituted arylsilyl group having 8 to 30 ring carbon atoms, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon atoms, or a substituted or unsubstituted heterocyclic group having 5 to 30 ring atoms; and Ar¹² and Ar¹³ each represent a substituted or unsubstituted aryl group having 6 to 30 ring carbon atoms, or a substituted or unsubstituted heterocyclic group having 5 to 30 ring atoms.
